(19) Europäisches Patentamt
European Patent Office
Office européen des brevets

(11) Publication number: **0 499 015 A1**

# (12) EUROPEAN PATENT APPLICATION

(21) Application number: **91850155.2**

(51) Int. Cl.[5]: **C07D 311/32**, A61K 31/35

(22) Date of filing: **07.06.91**

(30) Priority: **15.02.91 US 656801**

(43) Date of publication of application: **19.08.92 Bulletin 92/34**

(84) Designated Contracting States: **AT BE CH DE DK ES FR GB GR IT LI LU NL SE**

(71) Applicant: **Fockerman, Jasmine**
**PL 862**
**S-260 70 Ljungbyhed(SE)**
Applicant: **Fockerman, Michel**
**Styrmansgatan 23**
**S-114 54 Stockholm(SE)**

(72) Inventor: **Markonius, Maria**
**Byälvsvägen 139**
**S-121 74 Johanneshov(SE)**

(74) Representative: **Fagerlin, Heléne et al**
**H. ALBIHNS PATENTBYRA AB P.O. Box 3137**
**S-103 62 Stockholm(SE)**

## (54) Benzopyran phenol derivates for use as antibacterial, antiviral or immunostimulating agents.

(57) The invention concerns compounds with the general formula I

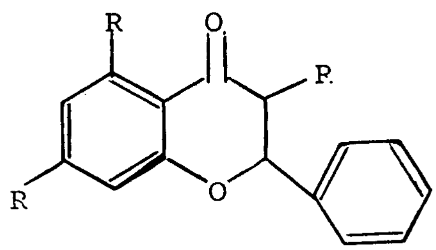

where R is the same or different and represents H, OH or

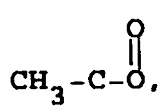

or mixtures thereof for use as an antibacterial, antiviral or immunostimulating agent or blood coagulating factor, a composition and a process for preparing such a mixture.

In the process an alcoholic solution containing propolis is added to a water solution containing 0.1-17 weight % of NaCl with a temperature of 30-95 °C, and the mixture is kept at 30-95 °C for 10-20 hours, whereafter the solution is freed from the bottom sediment.

EP 0 499 015 A1

The present invention concerns benzypyran phenol derivates or mixtures thereof for use as antibacterial, antiviral or immunostimulating agents, a process for preparing a mixture of such derivates and a composition for human or veterinary use containing at least one of the derivates.

The benzopyran phenol derivates according to the invention can be derived from propolis. The ethanol extract of propolis can be used as profylaxis for and against inflammations caused by certain viral infections (influenza, herpes) and is used as an antiinflammatory agent.

Propolis, also known as bee glue, is a natural product of bees. They collect it on the buds and other parts of plants, using it in their habitat to block up holes and cracks and also to isolate foreign bodies (insects and other living creatures) in the hive, thus preventing the spread of infections. They use it to coat the cells of the honeycomb before storing their products such as honey and pollen in them. Propolis is a specific complex bioactive substance consisting of more than 60 compounds. The basic components are different resins, waxes, ethereal oils and pollen. In addition, major bioactive ingredients of propolis are vegetable dyes, of which the most important are the flavones or yellow dyes such as chrysin or tectochrysin, flavonones such as pinostrombin, pinocembrin and quercetin and their derivatives, and also flavonols such as rhamnocitrin, galangin and isoalpinin. It also contains aroma substances such as isovanillin and acetoxybetulinol, and aromatic acids such as cinnamic acid, benzoic acid, caffeic acid, ferulic acid and protocatechuic acid with their esters with benzyl alcohol, pentanol, phenylethyl alcohol and cinnamyl alcohol (E M Schneidewind, A Brige, H Kala, J Metzner and A Zsunke, in Die Pharmazie No. 34, 1979, 103). Most of the substances listed exert an antimicrobial and fungistatic effect, which is also characteristic of the action of propolis.

It has now turned out that the compounds with the general formula I

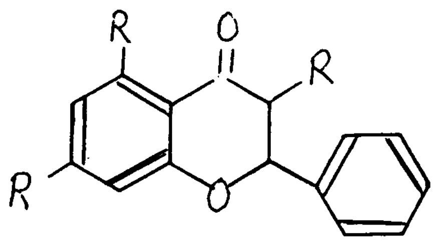

in which R is the same or different and represents H, OH or

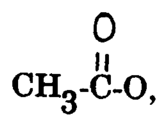

are components in propolis that can be used as antibacterial, antiviral or immunostimulating agents, which agents have better effects than propolis.

The invention especially concerns the use of Pinocembrin, 4H-1-bensopyran-4-on, 2,3-dihydro-5,7-dihydroxy-2-phenyl and Pinobanksin-3-acetate, 4H-1-bensopyran-4-on, 2,3-dihydro-5,7-dihydroxy-2-phenyl-3-acetate, for use as an antibacterial, antiviral or immunostimulating agent.

The invention also concerns mixtures of compounds with the general formula I and especially mixtures of Pinicembrin and Pinobanksin-3-acetate.

According to the present invention there is used an alcoholic solution containing propolis. This alcoholic solution is added to a water solution containing 0.1-17 weight % of NaCl with a temperature of 30-95 °C, and the mixture is kept at 30-95 °C for 10 to 20 hours, whereafter the solution is freed from the bottom sediment.

The alcohol may be an alcohol containing 1-20 carbon atoms, preferably 1-5 carbon atoms, such as methanol, ethanol, propanol, butanol, especially ethanol. Preferably there is used an ethanolic propolis solution prepared according to German patent 88109824.8.

According to this patent a mixture of the substances with formula I can be prepared from a propolis extract by extracting propolis in a closed system at a low temperature, 0-20 °C with an ethanol/water mixture at a volume ratio of 87 : 13 under ultrasonic treatment from 18 to 25 kHz for a short period such as 25 minutes, the suspension obtained then being decanted and the clear propolis extract freed from the solid

particles. Preferably the propolis is ground to a particle size of 3 mm diameter before extraction. The propolis is ground in a suitable mill into fine particles (max. diameter 5 mm). The German Patent No. 88109824.8 is hereby incorporated as a reference.

The alcoholic solution is preferably added to the NaCl-solution in an amount of 1-60 % by volume counted on the sum of the volumes of the two solutions.

The propolis solution is preferably added to the NaCl-solution drop by drop over about 1-7 hours, preferably 5 hours. A brown sediment is formed. The mixture is kept at 30-95 ° C for another 5-20 hours, preferably about 15 hours. A light yellow solution is formed over the hard dark brown sediment.

The substances and the compositions according to the invention can be used as antimicrobial agents for human or veterinary use. They can be used for profylaxis or treatment of inflammations or infections caused by gram positive or negative bacterias, viruses and funguses. The substances or mixtures thereof can be used as fodder, food-stuffs, hygienic articles, medicines and nature medicines. They can also be used as immunostimulating agents, such as adjuvants, and as blood-coagulation factors.

They can also be used for preparing containers and tools for fodder and food-stuffs and for treatment of surgical instruments, dental instruments, cosmetic tools such as syringes, bandages, plasters, dressings, compresses, rinsing solutions. Further they can be used for treating all sorts of space such as operating rooms, rooms where animals are kept, places for preparing or storing foods and fodder. Having immune stimulating activity they can also be used in vaccines as adjuvants.

When using the extraction process according to the invention there is obtained a sodium chloride solution containing Pinocembrin and Pinobanksin-3-acetate. The extract can be adjusted to contain preferably a physiological solution of 0.6-0.9, especially 0.9 % NaCl. This is very suitable for treating or preventing mastitis i.e. inflammation in the udder. The saline extract could be prepared under sterile conditions and be injected directly in the udder. "Provet" which is used today contains crude propolis (from Apipharm Co. Ltd.), Lanacolum, alcohol-cetyle-stearate, polyoxyethylene-sorbitan-monolaurate, vaseline and paraffin. This product "Provet" must be introduced in the milk canal of each teat of the diseased mammal at the end of milking "Provet" thus consists of four disposable syringes for injection in each of the teats. Thus the composition according to the invention is easier to administer.

The immunostimulating effect of the mixture according to the invention has been tested directly on cells from thymus and spleen (B- and T-lymphocytes and natural killer cells (NK cells)). In these tests the mixture showed potent immunostimulating effects. The composition according to the invention has also been given to animals in their feed giving rise to increased activity of T- and B-cells in both thymus and spleen. The composition according to the invention increases both the humoral and cell mediated immune response.

In mice infected with Coxsackievirus B3 (CB3) the lifetime was significantly prolonged for mice treated prophylactically with the composition.

When tested as 1-10 % nose spray in a total of 58 patients no local or systemic allergic reactions were observed.

The substances and the compositions according to the invention can be used in any pharmaceutical form such as tablets, capsules, solutions for injections, solutions or spray for nasal treatment.

The administration forms may contain pharmaceutically acceptable carriers such as one or more compatible solid filler diluents or solid or liquid substances added to aid in the production of the pharmaceutical forms, such as lubricants to reduce friction and glidants to improve flow of the particulate mixtures. By "compatible" as used herein, is meant that the components are capable of being comingled without interacting in a manner which would substantially decrease the pharmaceutical efficacy.

Some examples of substances which can serve as pharmaceutical carriers are sugars, such as lactose, glucose and sucrose; starches, such as corn starch and potato starch; cellulose and its derivatives, such as sodium carboxymethylcellulose, ethylcellulose, cellulose acetate; powdered tragacanth; malt; gelatin; talc; stearic acid; magnesium stearate; zinc stearate; calcium sulphate; silicon dioxide; vegetable oils, such as peanut oil, cottonseed oil, sesame oil, olive oil, corn oil and oil of theobroma; polyols, such as propylene glycol, glycerine, sorbitol, mannitol, and polyethylene glycol; agar; and alginic acid; as well as other non-toxic compatible substances used in pharmaceutical formulations. Wetting agents such as sodium laural sulphate, as well as coloring agents, lubricants, excipients, stabilizers, antioxidants, and preservatives, can also be present.

For nasal treatment there can be used a physiological NaCl-solution containing 0.9 % by weight NaCl and 1-20 % propolis extract. For treatment of mastitis there can be used a 0.9 % NaCl-solution containing 20-50 % propolis extract, preferably 30 %.

When talking about % propolis extract in this application there is meant the volume of the starting alcoholic propolis solution in relation to the sum of the propolis and NaCl-solutions. Thus the end product of

Example 2 is considered to contain 30 % propolis extract.

Test of the bactericidal effects of the extracts according to examples 2 and 5.

The following strains were tested: Klebsiella oxytoca, Pseudomonas aeruginosa, koagulasnegativ stafylokock, Streptococcus uberis, Streptococcus agalactiae, Proteus mirabilis, Actinomyces pyogenes, Saccaromyces cerevisae, Candida pseudo-tropicalis (Kluyveromyces marxianus).

The four propolis solutions were negative at sterility control tests made before the growth tests. All strains were tested once. They were cultivated in bovine broth at 37°C during 20 hours whereafter the broths contained $10^6$-$10^9$ microorganisms per ml (see table I).

Samples of 0.1 ml were taken from each broth (dilution $10^2$ or $10^3$) and added to 0.9 ml of the extract according to example 2 or 5 so as to make each mixture contain $10^2$-$10^5$ bacteria/ml (see table II). The solutions were then incubated at 38°C. After 0 and 3 hours the bacteria were counted. The results are given in tables III-IV. Alter cultivation 3 hours there were no bacteria in the culture, which indicates that every bacterial group tested was dead after incubation three hours in the tested extracts according to Examples 2 and 5.

Table I

| Number of bacteria in the broths | |
|---|---|
| Test 1 | Test 2 |
| Ex 2 | Ex 5 |
| Kl 1.5*$10^9$ | 2.0*$10^9$ |
| P.a 4.0*$10^9$ | 12*$10^9$ |
| SK- 5.5*$10^9$ | 5.0*$10^9$ |
| Sru 9.5*$10^8$ | 3.5*$10^8$ |
| Sra 4.0*$10^7$ | 9.5*$10^7$ |
| P.m 2.5*$10^9$ | 2.0*$10^9$ |
| A.p 8.5*$10^9$ | 1.0*$10^9$ |
| S.ce 1.5*$10^7$ | 3.0*$10^6$ |
| C.ps 3.5*$10^7$ | 1.7*$10^7$ |

Table II

| Bacteria/ml extract | |
|---|---|
| Kl 1.5*$10^5$ | 2.0*$10^5$ |
| Pa 4.0*$10^5$ | 12*$10^5$ |
| SK- 5.5*$10^5$ | 5.0*$10^5$ |
| Sru 9.5*$10^5$ | 3.5*$10^5$ |
| Sra 4*$10^4$ | 9.5*$10^4$ |
| P.m 2.5*$10^5$ | 2.0*$10^5$ |
| A.p 8.5*$10^5$ | 1.0*$10^5$ |
| S.ce 1.5*$10^3$ | 3.0*$10^2$ |
| C.ps 3.5*$10^3$ | 1.7*$10^3$ |

Table III

| time 0h | | | |
|---|---|---|---|
| Ex 2 | Ex 5 | Ex 5 | Ex 6 |
| Kl $2.5*10^3$ | $7.5*10^4$ | $5.5*10^4$ | 0 |
| P.a 0 | 0 | 0 | $5.0*10^2$ |
| SK- $1*10^3$ | $2.0*10^3$ | $9.0*10^3$ | $8.0*10^3$ |
| Sru 0 | 0 | $3.0*10^3$ | $5.0*10^2$ |
| Sra 0 | $1.0*10^3$ | $5.0*10^2$ | 0 |
| P.m $2*10^5$ | $9.5*10^4$ | $5.5*10^4$ | 0 |
| A.p 0 | 0 | $1.0*10^5$ | $1.0*10^4$ |
| S.ce 0 | 0 | $2.5*10^2$ | $1.0*10^2$ |
| C.ps $1*10^3$ | $3.0*10^3$ | $1.1*10^3$ | $1.5*10^3$ |

Table IV

| time 3h | | | |
|---|---|---|---|
| Kl 0 | 0 | 0 | 0 |
| P.a 0 | 0 | 0 | 0 |
| SK- 0 | 0 | 0 | 0 |
| Sru 0 | 0 | 0 | 0 |
| Sra 0 | 0 | 0 | 0 |
| P.m 0 | 0 | 0 | 0 |
| A.p 0 | 0 | 0 | 0 |
| S.ce 0 | 0 | 0 | 0 |
| C.ps 0 | 0 | 0 | 0 |

Control: 10 ml of the propolis bacterial suspension was added to 3 ml broth and cultivated at 37° C for 24 hours and transferred to a plate and cultivated 48 hours at 37° C. The growth was 0.

The following three strains were analyzed in another test: Staphylococcus aureus, Streptococcus dysgalactiae, Escherichia coli.

All propolis solutions were checked for existence of bacteria. They were all free from bacteria.

The bacteria were cultivated in broth for 24 hours. They then contained $10^7$-$10^8$ bacteria per ml. From every broth culture 0.1 ml was taken out and mixed with 0.9 ml of propolis sulution and incubated at 37° C. Bacteria were counted alter 0, 3, 24 and 48 hours.

Result: No bacteria could be found in the cultures after cultivating during 3 hours which indicates that all bacteria were dead.

The following examples illustrate the invention without limitating it in any way.

Example 1 (Preparation of the starting material)

In the extraction vessel, 500 litres of an ethanol solution not less than 87 % by weight is prepared. While the mixture is being stirred, 20 kg of the ground propolis is added to the contents of the extractor. The batch is subjected to ultrasonic extraction (18 - 25 kHz) for 25 minutes, then left to stand for 2 hours, decanted and filtered.

The filtration is effected via a pressure filter with rapid filter-paper inserts. The clear propolis extract obtained is reduced to the required concentration of 5 to 80 % of the dry substance in a column concentrator in a 150 - 50 mm $H_2O$ vacuum by means of a heat pump. The concentration is performed at a temperature of max. 20° C.

Example 2

The extract obtained in example 1 was diluted to contain a dry substance weight of 10 % in ethanol. NaCl was dissolved in distilled water to a concentration of 0.9 %. 700 ml of the solution was placed in a

beaker which was put into a water bath. The temperature in the beaker was kept at 30 ° C. 300 ml of the propolis extract containing 10 weight % propolis in ethanol was added drop by drop to the NaCl colution during about 5 hours. A muddy brown precipitate was obtained. The temperature of the solution was controlled by keeping the water bath boiling and by continuously adding water during 15 hours to keep the level. By then a light yellow solution was formed the volume of which was 700 ml. The rest of the solution had evaporated during the heating. The beaker was left in the water bath until the content had reached room temperature. The precitipate was freed from the solution. Analysis by mass spectra of the yellow solution shows that it contains Pinocembrin and Pinobanksin-3-acetate.

Example 3

The extract obtained in Example 1 was diluted to contain a dry substance weight of 10 % in ethanol. NaCl was dissolved in distilled water to a concentration of 10 %. 700 ml of the solution was placed in a beaker which was put into a wather bath. The temperature in the beaker was kept at 30 ° C. 300 ml of the propolis extract containing 10 weight % propolis in ethanol was added drop by drop to the NaCl colution during about 5 hours. A muddy brown precipitate was obtained. The temperature of the solution was controlled by keeping the water bath boiling and by continuously adding water during 15 hours to keep the level. By then there was formed a light yellow solution the volume of which was 700 ml. The rest of the solution had evaporated during the heating. The beaker was left in the water bath until the content had reached room temperature. The precitipate was freed from the solution.

Example 4

The extract obtained in example 1 was diluted to contain a dry substance weight of 10 % in ethanol. NaCl was dissolved in distilled water to a concentration of 15 %. 700 ml of the solution was placed in a beaker which was put into a water bath. The temperature in the beaker was kept at 30 ° C. 300 ml of the propolis extract containing 10 weight % propolis in ethanol was added drop by drop to the NaCl colution during about 5 hours. A muddy brown precipitate was obtained. The temperature of the solution was controlled by keeping the water bath boiling and by continuously adding water during 15 hours to keep the level. By then there was formed a light yellow solution the volume of which was 700 ml. The rest of the solution had evaporated during the heating. The beaker was left in the water bath until the content had reached room temperature. The precitipate was freed from the solution.

Example 5

The procedure of example 2 was repeated but there was used instead 800 ml of the NaCl solution and 200 ml of the propolis extract.

Example 6

The procedure of example 2 was repeated but there was used instead 770 ml of the NaCl solution and 230 ml of the propolis extract.

Example 7

The procedure of example 2 was repeated but there was used instead 500 ml of the NaCl solution and 500 ml of the propolis extract.

The light yellow solution was further purified by dialysis against water. 50 ml of the extract was dialysed against 250 ml of destilled water through a tube (mw cataf 3500 Kebo AB Sweden).

Both the undialysed and the dialysed solution were tested by mass spectra with Solid Probe/µs. 1 µl of a mixture of the test solution and 96 % p.a. EtOH 1:1 was evaporated at about 60 ° C, chilled to 25 ° C and then heated 10 ° C/s to 375 ° C which temperature was maintained for about 4 min. Mass spectra from m/2 41-641 with cycle 1s was taken during this period. The mass spectra of the dialysed product are shown in figure 1 of which fig. 1a shows the temperature ramp, the four regions A-D and the total ion stream and fig. 1b shows the total ion stream after a numerical filter. Figure 1c shows the spectrum in the region A, figure 1d the spectrum of region B, figure 1e the spectrum of region C and 1f the spectrum of region D respectively. Figures 2a - 2f show the same spectra for the undialysed product. The mass spectra are interpreted to show that the product contains Pinocembrin and Pinobanksin-3-acetate.

## Claims

1. Compounds with the general formula I

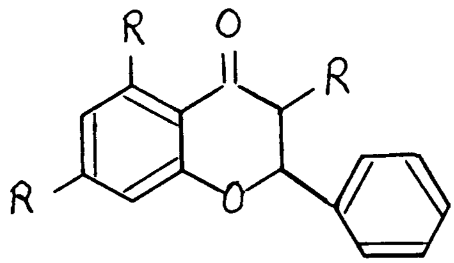

where R is the same or different and represents H, OH or

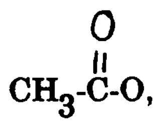

for use as an antibacterial, antiviral or immunostimulating agent or blood coagulating factor.

2. Pinocembrin 4H-1-Bensopyran-4-on, 2,3-dihydro-5,7-dihydroxy-2-phenyl according to claim 1 for use as an antibacterial, antiviral, immunostimulating agent or blood-coagulating factor

3. Pinobanksin-3-acetate, 4H-1-bensopyran-4-on, 2,3-dihydro-5,7-dihydroxy-2-phenyl-3-acetate according to claim 1 for use as an antibiotic, antiviral or immunostimulating agent or blood coagulating factor.

4. A mixture of the compounds according to claim 1 for use as an antibiotic, antiviral or immunostimulating agent or blood coagulating factor.

5. A mixture of Pinobanksin-3-acetate and Pinocembrin for use as an antibiotic, antiviral or immunostimulating agent or blood coagulating factor.

6. A process for preparing a mixture according to claim 4, whereby an alcoholic solution containing propolis is added to a water solution containing 0.1-17 weight % of NaCl with a temperature of 30-95 °C, and the mixture is kept at 30-95 °C for 10-20 hours, whereafter the solution is freed from the bottom sediment.

7. A process according to claim 6, wherein the alcoholic propolis solution is a solution obtained by extracting propolis in a closed system at a low temperature, e.g. max. 20 °C with an ethanol/water mixture at a volume ratio of 87 : 13 under ultrasonic treatment from 18 to 25 kHz for a short period such as 25 minutes, the suspension obtained then being decanted and the clear propolis extract freed from the solid particles.

8. A composition for human or veterinary use containing at least one of the substances according to claims 1-5 or produced according to claim 6 or 7 in pharmaceutical acceptable carrier.

9. A composition according to claim 8, wherein the carrier is a physiological saline solution.

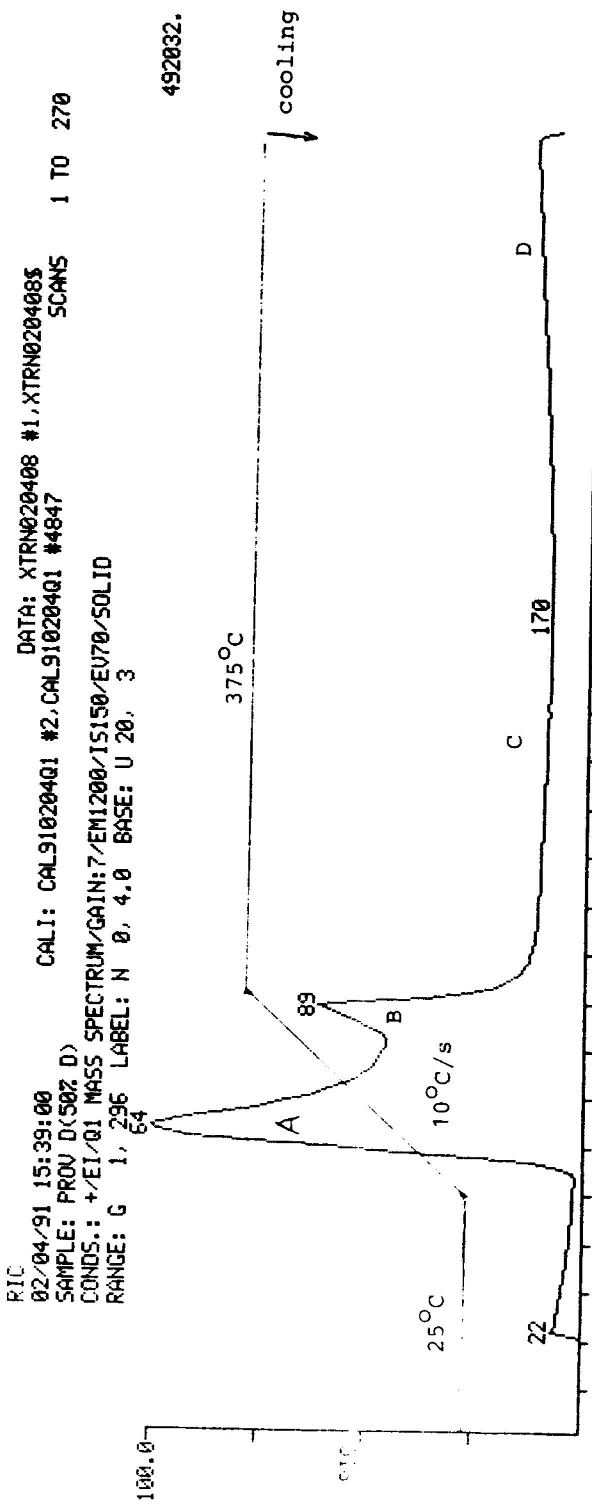
RIC
DATA: XTRN020408 #1,XTRN020408S
02/04/91 15:39:00 CALI: CAL910204Q1 #2,CAL910204Q1 #4847 SCANS 1 TO 270
SAMPLE: PROV D(50% D)
CONDS.: +/EI/Q1 MASS SPECTRUM/GAIN:7/EM1200/IS150/EV70/SOLID
RANGE: G 1, 296 LABEL: N 0, 4.0 BASE: U 20, 3
492032.
cooling
375°C
10°C/s
25°C
A
B
C
D
22
64
89
170
100.0

FIG. 1a

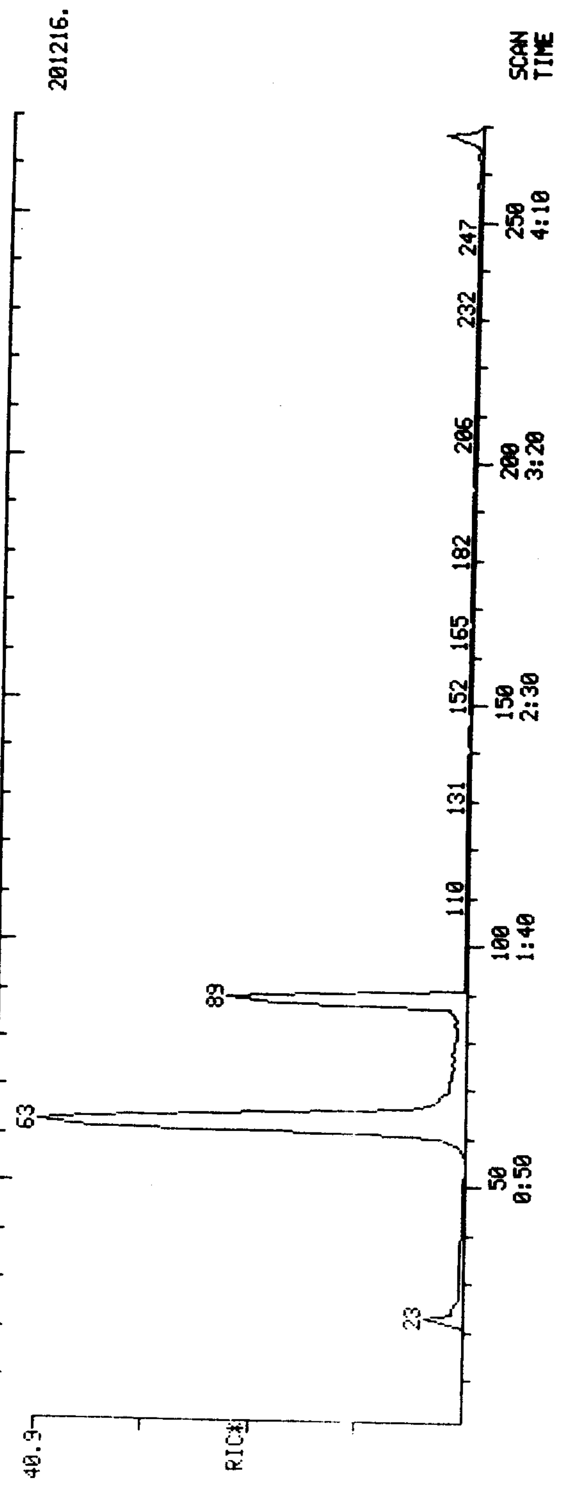
201216.
40.9
RIC
23
63
89
110
131
152
165
182
206
232
247
50
0:50
100
1:40
150
2:30
200
3:20
250
4:10
SCAN
TIME

FIG. 1b

FIG. 1c

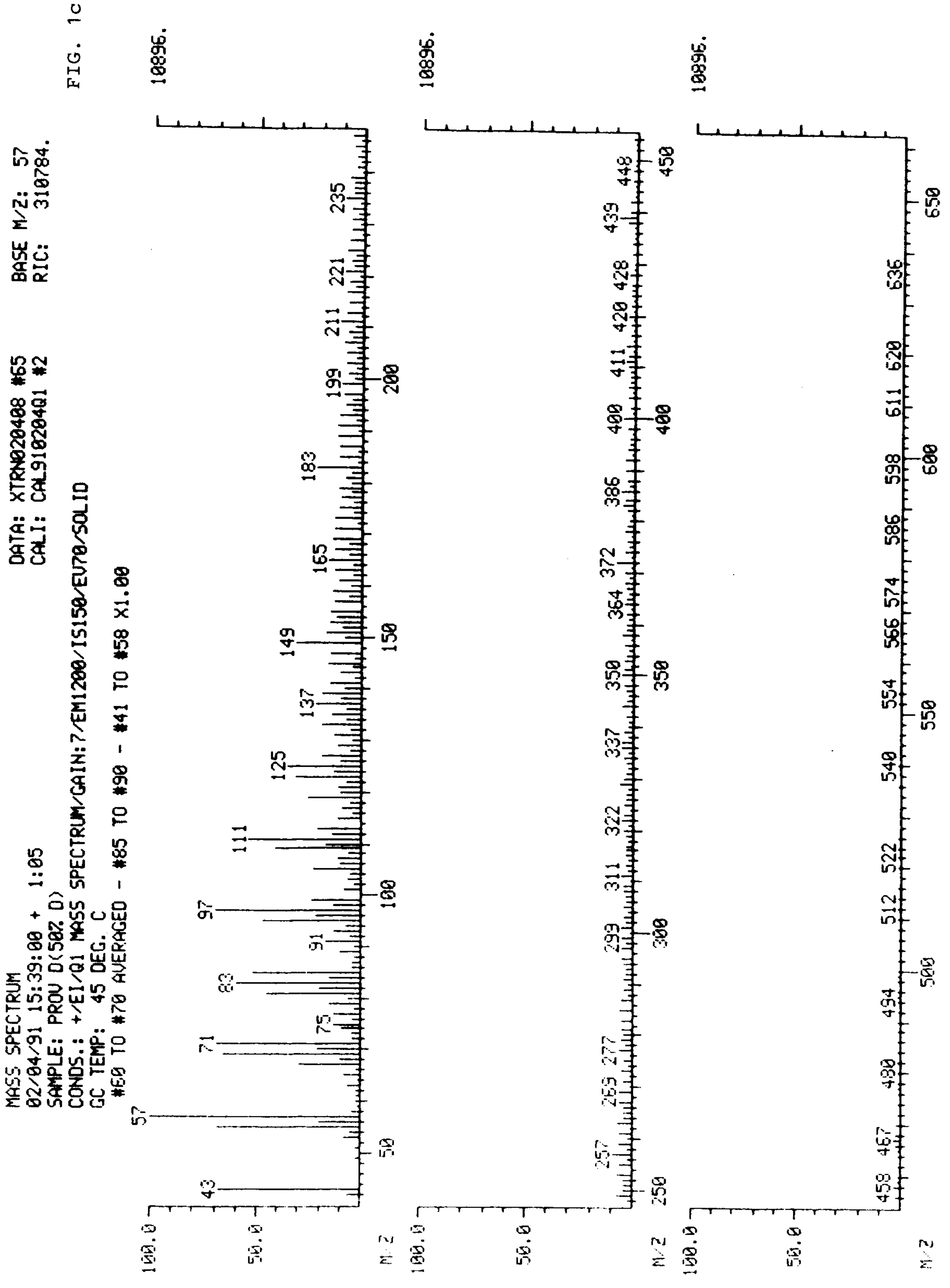
MASS SPECTRUM
02/04/91 15:39:00 + 1:05
SAMPLE: PROV D(50% D)
CONDS.: +/EI/Q1 MASS SPECTRUM/GAIN:7/EM1200/IS150/EV70/SOLID
GC TEMP: 45 DEG. C
#60 TO #70 AVERAGED - #85 TO #90 - #41 TO #58 X1.00
DATA: XTRN020408 #65
CALI: CAL910204Q1 #2
BASE M/Z: 57
RIC: 310784.
10896.
100.0
50.0
M/Z

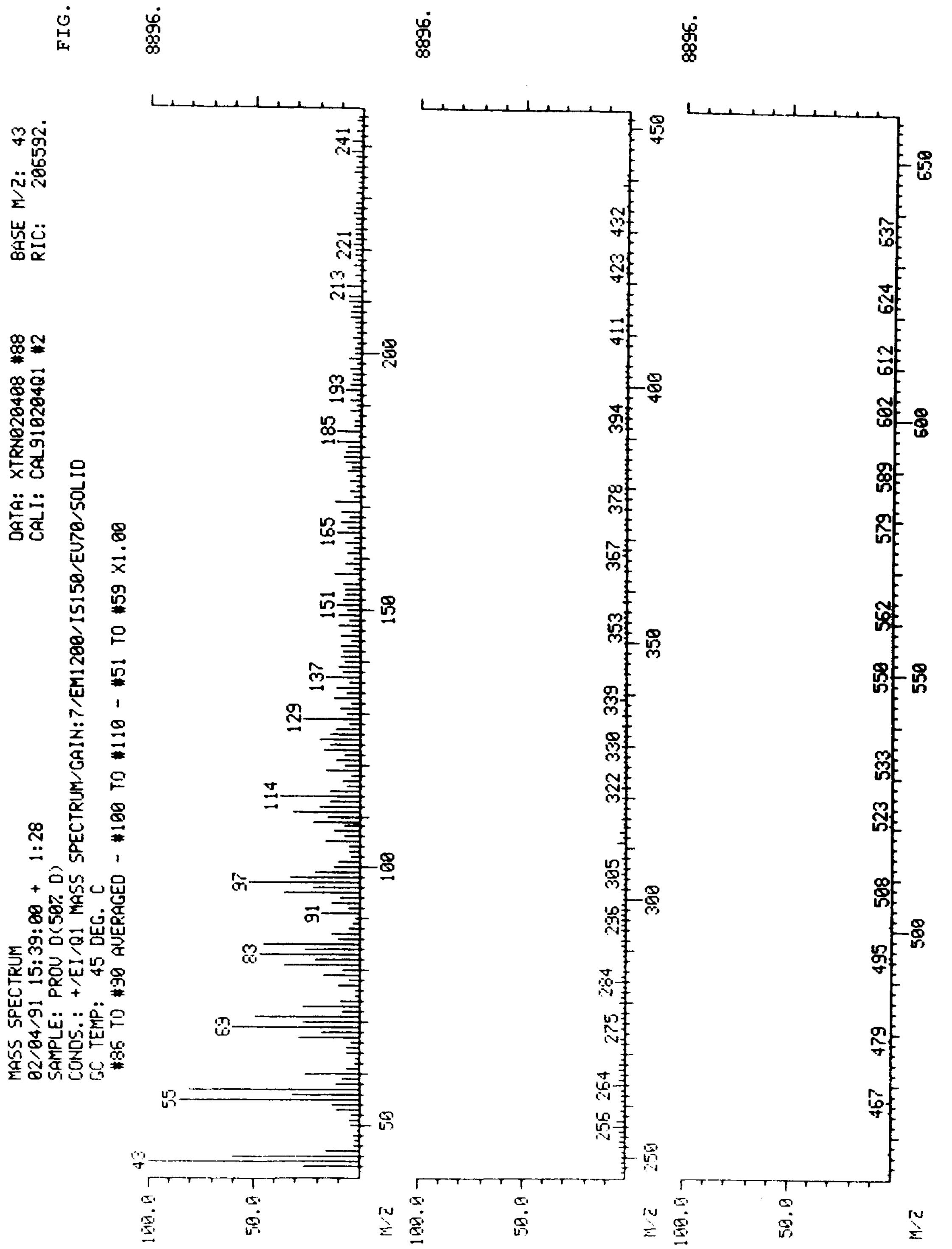
MASS SPECTRUM
02/04/91 15:39:00 + 1:28
SAMPLE: PROV D(50% D)
CONDS.: +/EI/Q1 MASS SPECTRUM/GAIN:7/EM1200/IS150/EV70/SOLID
GC TEMP: 45 DEG. C
#86 TO #90 AVERAGED - #100 TO #110 - #51 TO #59 X1.00
DATA: XTRN020408 #88
CALI: CAL910204Q1 #2
BASE M/Z: 43
RIC: 206592.
8896.
100.0
50.0
M/Z

FIG. 1d

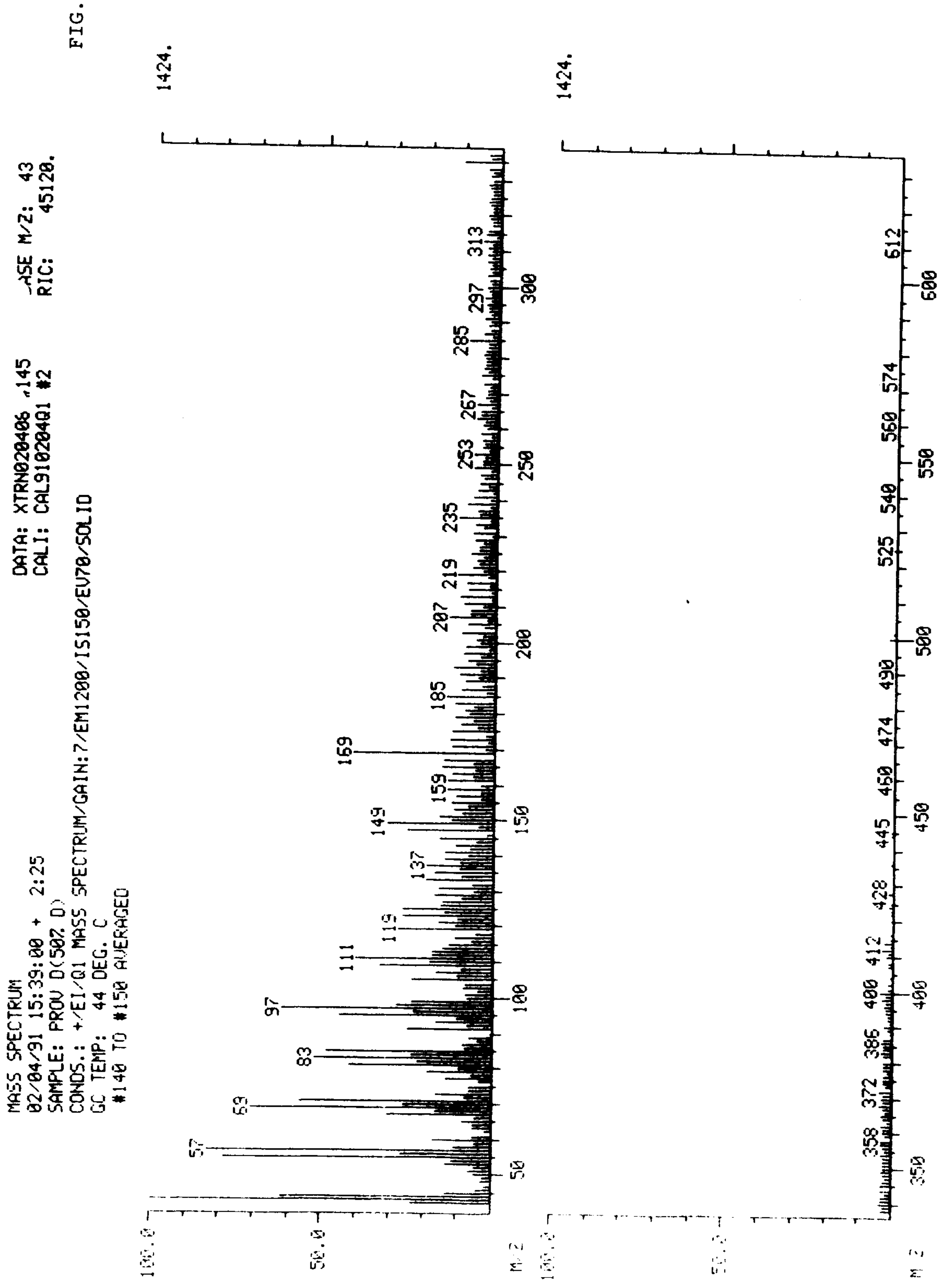
MASS SPECTRUM
02/04/91 15:39:00 + 2:25
SAMPLE: PROV D(50% D)
CONDS.: +/EI/Q1 MASS SPECTRUM/GAIN:7/EM1200/IS150/EV70/SOLID
GC TEMP: 44 DEG. C
#140 TO #150 AVERAGED
DATA: XTRN020406 #145
CALI: CAL910204Q1 #2
BASE M/Z: 43
RIC: 45120.
1424.
100.0
50.0
M/Z
57
69
83
97
111
119
137
149
159
169
185
207
219
235
253
267
285
297
313
50
100
150
200
250
300
358
372
386
400
412
428
445
460
474
490
525
540
560
574
612
350
400
450
500
550
600

FIG. 1e

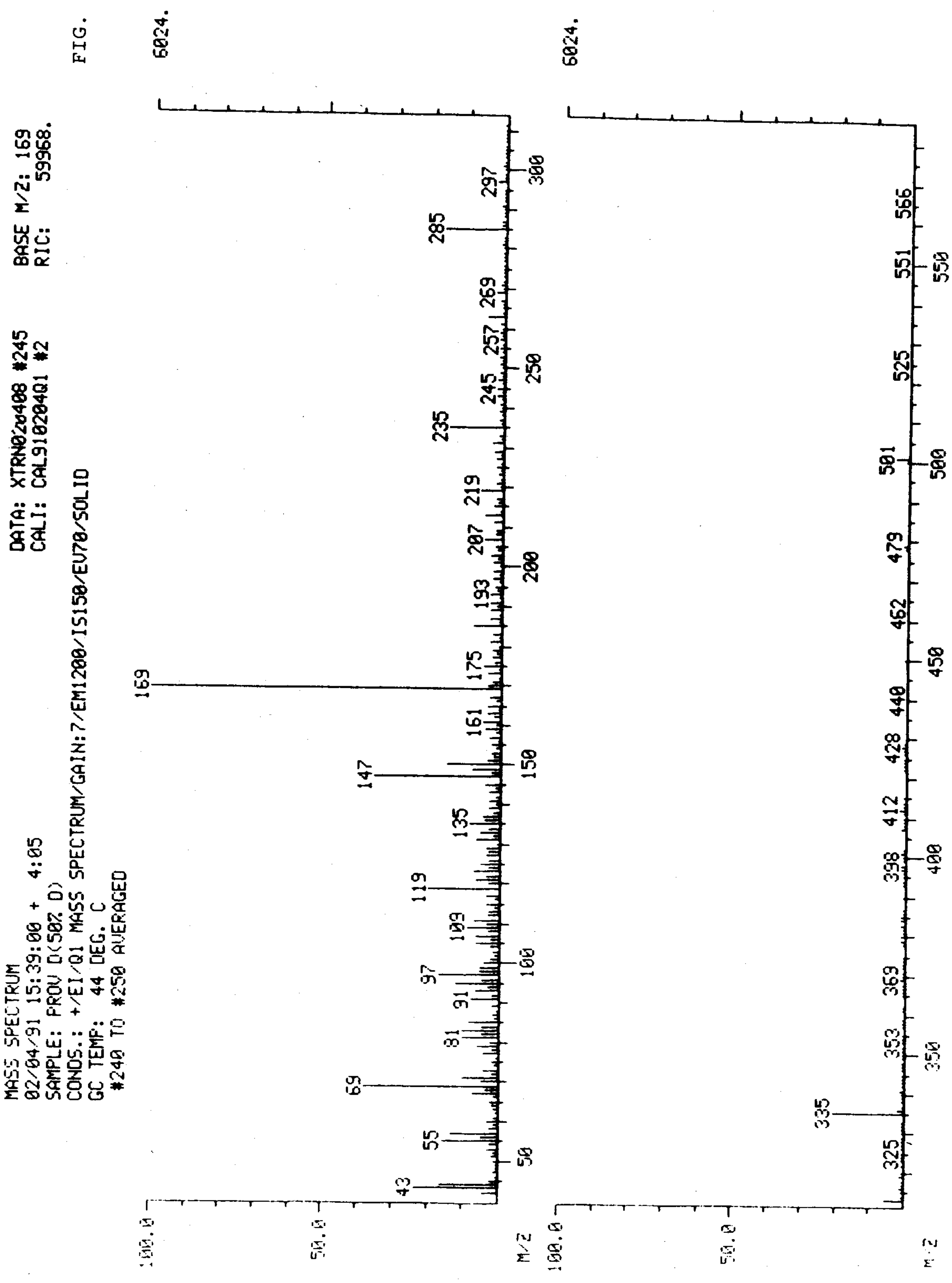
MASS SPECTRUM
02/04/91 15:39:00 + 4:05
SAMPLE: PROV D(50% D)
CONDS.: +/EI/Q1 MASS SPECTRUM/GAIN:7/EM1200/IS150/EV70/SOLID
GC TEMP: 44 DEG. C
#240 TO #250 AVERAGED
DATA: XTRN02d408 #245
CALI: CAL910204Q1 #2
BASE M/Z: 169
RIC: 59968.
6024.
M/Z

FIG. 1f

FIG. 2a

FIG. 2b

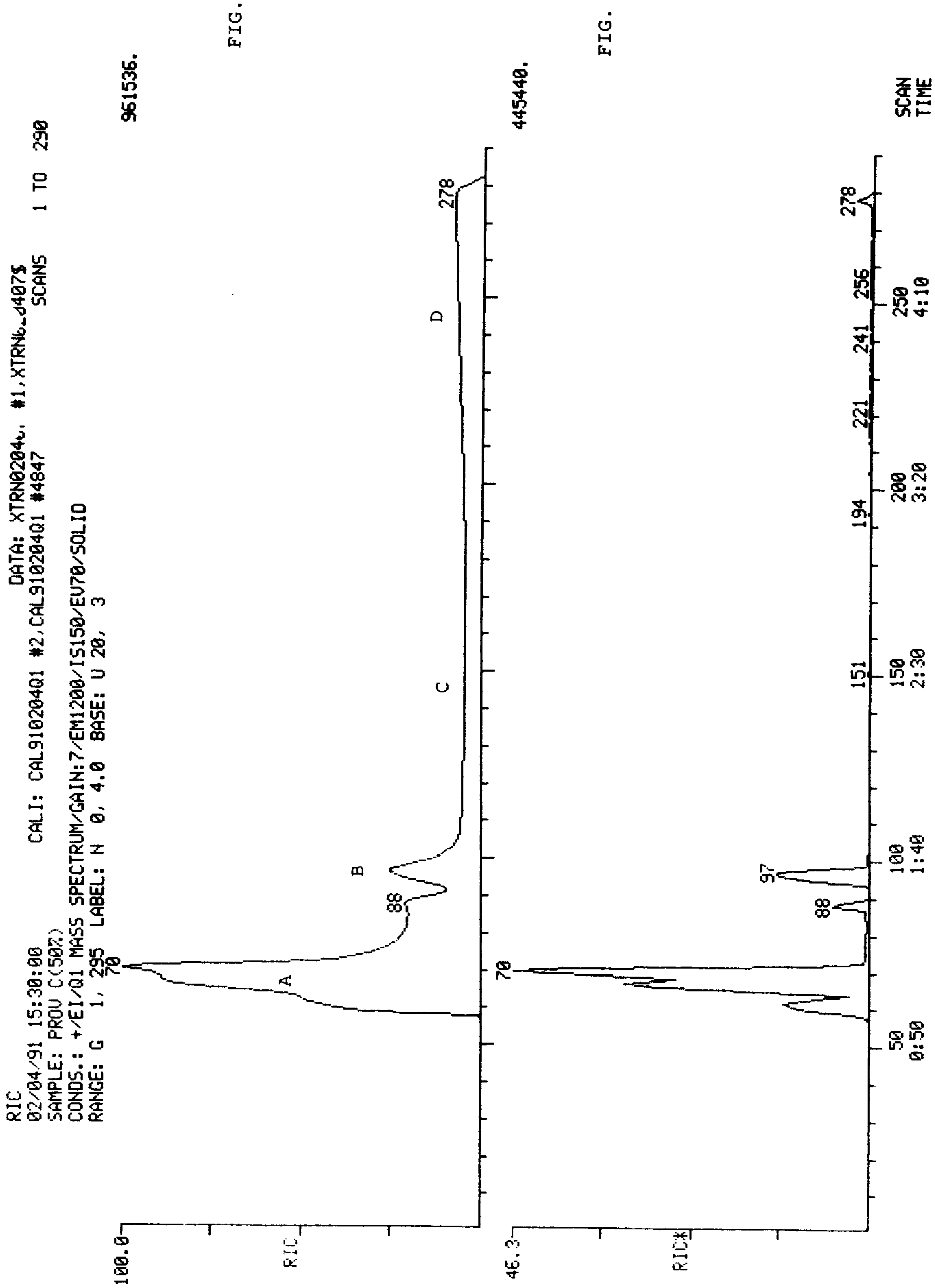
RIC
02/04/91 15:30:00
SAMPLE: PROV C(50%)
CONDS.: +/EI/Q1 MASS SPECTRUM/GAIN:7/EM1200/IS150/EV70/SOLID
RANGE: G 1, 295 LABEL: N 0, 4.0 BASE: U 20, 3
CALI: CAL910204Q1 #2,CAL910204Q1 #4847
SCANS 1 TO 290
961536.
445440.
100.0
RIC
46.3
RIC
A
B
C
D
70
88
278
97
151
194
221
241
256
50
0:50
100
1:40
150
2:30
200
3:20
250
4:10
SCAN
TIME

FIG. 2c

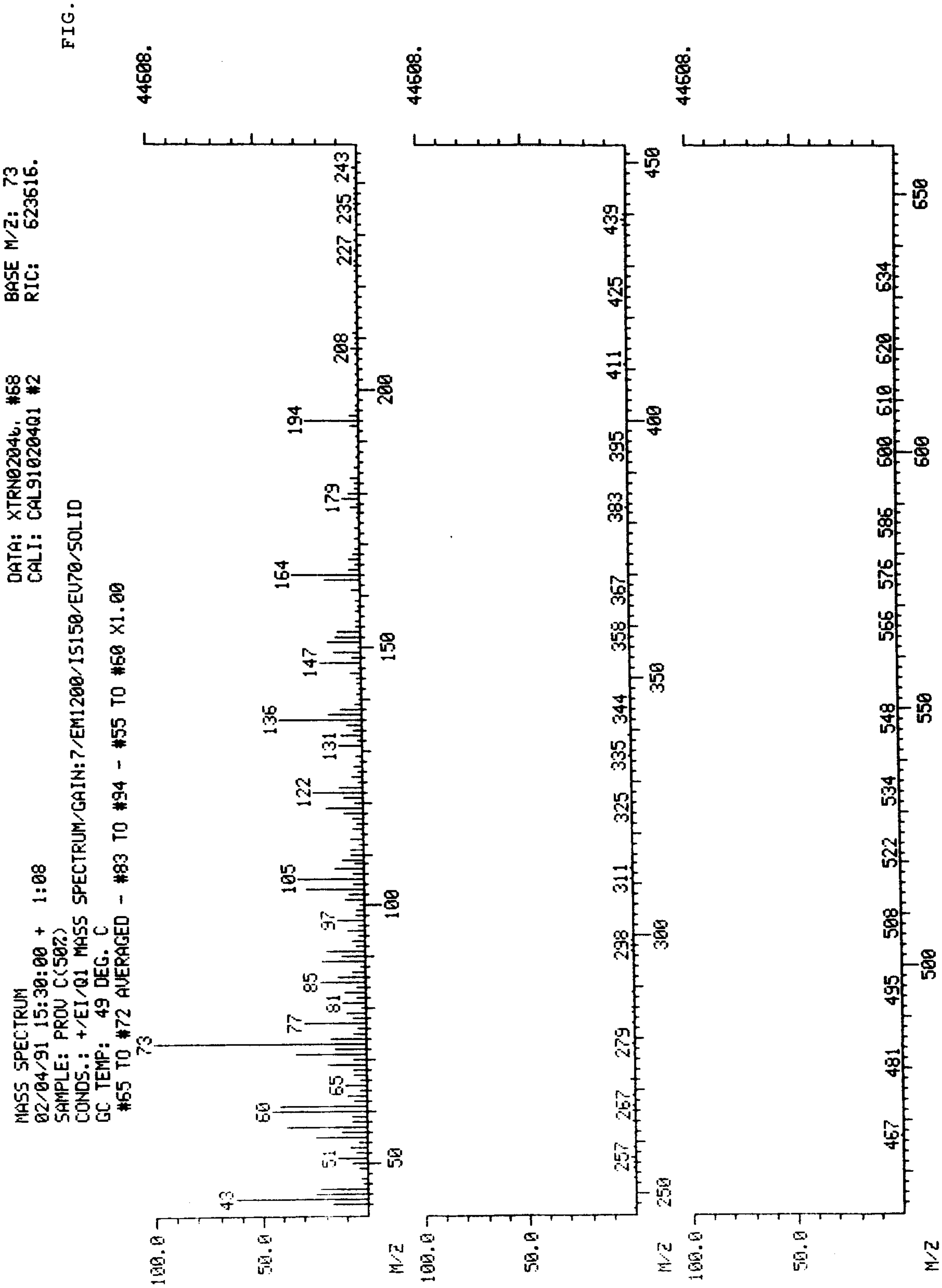
MASS SPECTRUM
02/04/91 15:30:00 + 1:08
SAMPLE: PROV C(50%)
CONDS.: +/EI/Q1 MASS SPECTRUM/GAIN:7/EM1200/IS150/EV70/SOLID
GC TEMP: 49 DEG. C
#65 TO #72 AVERAGED - #83 TO #94 - #55 TO #60 X1.00
DATA: XTRN02046. #68
CALI: CAL910204Q1 #2
BASE M/Z: 73
RIC: 623616.
44608.
100.0
50.0
M/Z

FIG. 2d

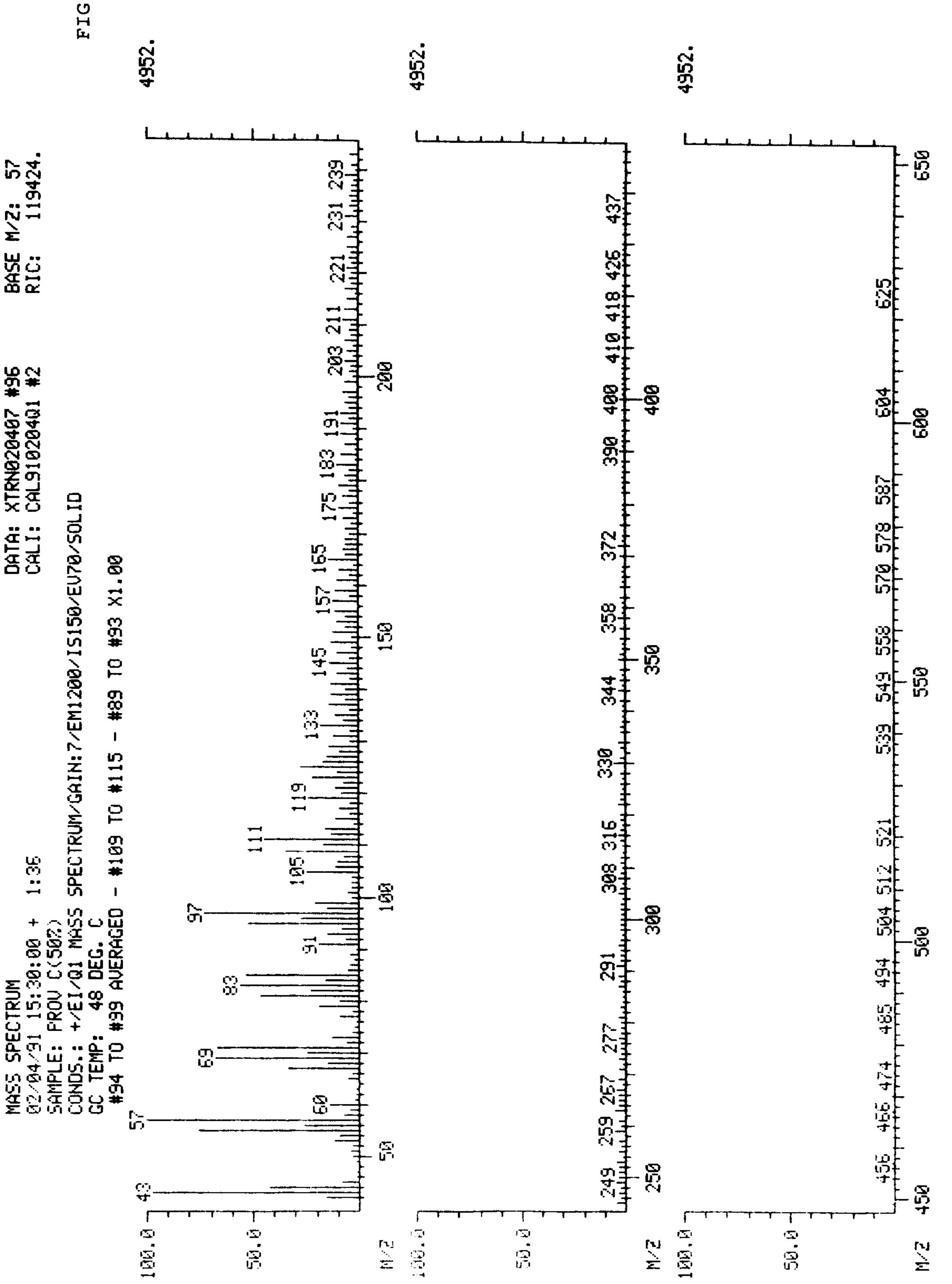
MASS SPECTRUM
02/04/91 15:30:00 + 1:36
SAMPLE: FROU C(50%)
CONDS.: +/EI/Q1 MASS SPECTRUM/GAIN:7/EM1200/IS150/EV70/SOLID
GC TEMP: 48 DEG. C
#94 TO #99 AVERAGED - #109 TO #115 - #89 TO #93 X1.00
DATA: XTRN020407 #96
CALI: CAL910204Q1 #2
BASE M/Z: 57
RIC: 119424.
4952.
100.0
50.0
M/Z

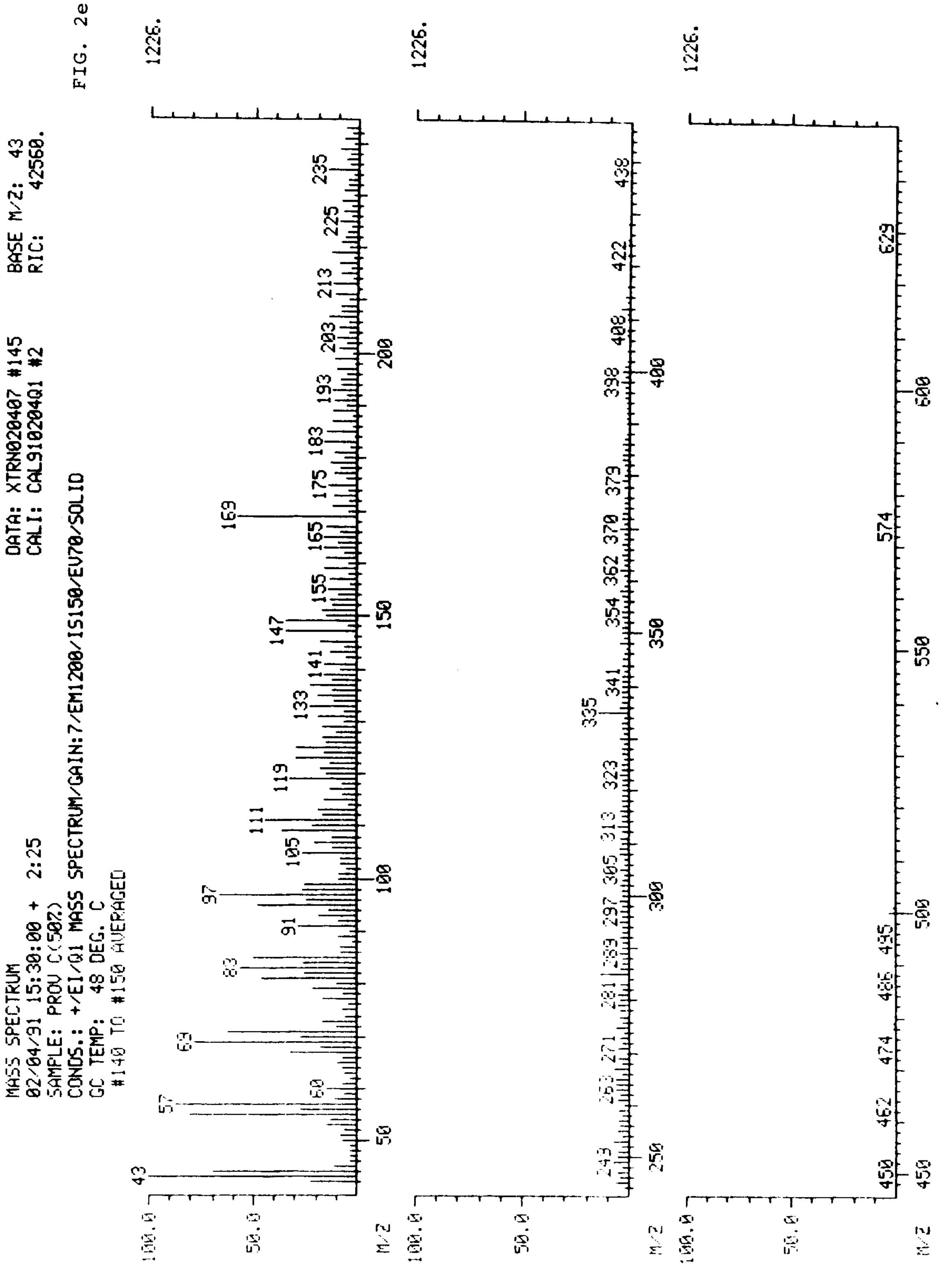
MASS SPECTRUM
02/04/91 15:30:00 + 2:25
SAMPLE: PROV C(50%)
CONDS.: +/EI/Q1 MASS SPECTRUM/GAIN:7/EM1200/IS150/EV70/SOLID
GC TEMP: 48 DEG. C
#140 TO #150 AVERAGED
DATA: XTRN020407 #145
CALI: CAL9102040Q1 #2
BASE M/Z: 43
RIC: 42560.
1226.
100.0
50.0
M/Z

FIG. 2e

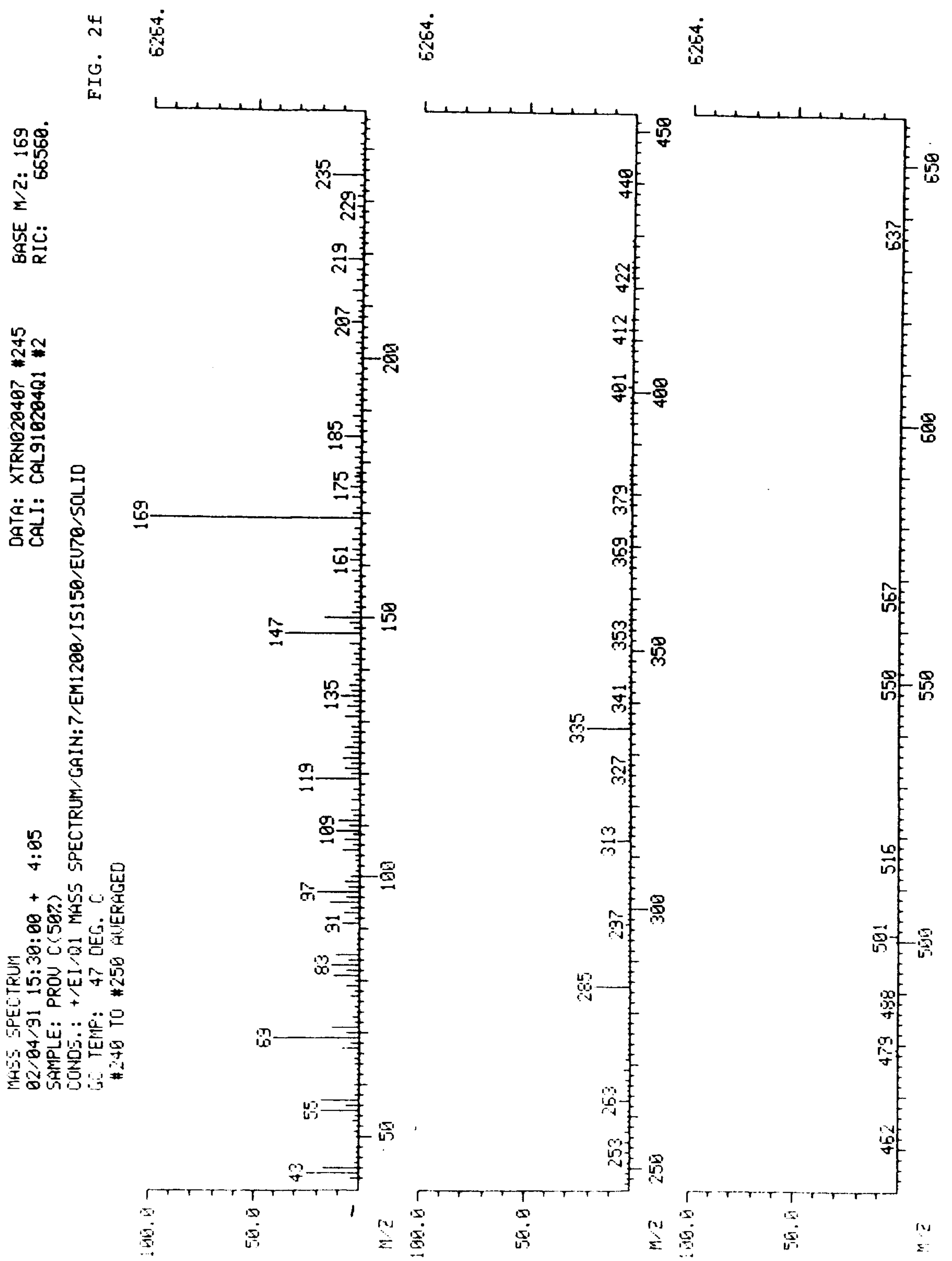
MASS SPECTRUM
02/04/91 15:30:00 + 4:05
SAMPLE: PROV C(50%)
CONDS.: +/EI/Q1 MASS SPECTRUM/GAIN:7/EM1200/IS150/EV70/SOLID
GC TEMP: 47 DEG. C
#240 TO #250 AVERAGED
DATA: XTRN020407 #245
CALI: CAL910204Q1 #2
BASE M/Z: 169
RIC: 66560.
6264.
M/Z
100.0
50.0

FIG. 2f

European Patent Office

# EUROPEAN SEARCH REPORT

Application Number

EP 91 85 0155
PAGE1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | WO-A-8 808 256 (UNIVERSITY PATENTS,INC)<br>* page 3, paragraph 3 - page 5 *<br>--- | 1 | C07D311/32<br>A61K31/35 |
| A | DE-A-1 793 025 (SOCIETE DE RECHERCHES INDUSTRIELLES S.O.R.I.S.A)<br>* page 1 - page 2, paragraph 3 *<br>--- | 1,8 | |
| A | PATENT ABSTRACTS OF JAPAN<br>vol. 11, no. 371 (C-462)(2818) 3 December 1987<br>& JP-A-62 145 016 ( MITSUBISHI CHEM.IND LTD. )<br>29 June 1987<br>* abstract *<br>--- | 1,8 | |
| X | PHYTOCHEMISTRY<br>vol. 26, no. 8, 23 July 1987, OXFORD<br>pages 2231 - 2234;<br>AKIHISA MORI ET AL.: 'Antibacterial activity and mode of action of plant flavonoids against Proteus Vulgaris and Staphylococcus Aureus'<br>* the whole document *<br>--- | 1,8 | TECHNICAL FIELDS SEARCHED (Int. Cl.5 ) |
| X | ACTA MICROBIOLOGICA BULGARICA<br>vol. 23, 1988, SOFIA<br>pages 52 - 57;<br>V.BANKOVA ET AL.: 'On the chemical composition of some propolis fractions with antiviral action'<br>* the whole document *<br>--- | 1,8 | C07D |
| X | LEBENSMITTEL-WISSENSCHAFT UND -TECHNOLOGIE<br>vol. 17, no. 2, 1984, SWITZERLAND<br>pages 74 - 76;<br>S.BOGDANOV: 'Characterisation of Antibacterial substances in Honey'<br>* the whole document *<br>---<br>-/-- | 1,8 | |

The present search report has been drawn up for all claims

| Place of search | Date of completion of the search | Examiner |
|---|---|---|
| BERLIN | 21 MAY 1992 | KYRIAKAKOU G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 91 85 0155
PAGE2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | AGRICULTURAL AND BIOLOGICAL CHEMISTRY<br>vol. 51, no. 1, 1987, TOKYO<br>pages 139 - 143;<br>CHIKAO NISHINO ET AL.: 'Antibacterial Activity of Flavonoids against Staphylococcus epidermidis,a Skin Bacterium'<br>* the whole document *<br>----- | 1,8 | |
| | | | TECHNICAL FIELDS SEARCHED (Int. Cl.5 ) |

The present search report has been drawn up for all claims

| Place of search | Date of completion of the search | Examiner |
|---|---|---|
| BERLIN | 21 MAY 1992 | KYRIAKAKOU G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document